# EUROPEAN PATENT APPLICATION

(11) **EP 2 135 688 A2**
(43) Date of publication of application: **23.12.2009**
(21) Application number: 08460045.1
(22) Date of filing: 27.11.2008
(51) Int. Cl.: B09B 3/00, B03B 9/06

(54) **The method and processing system for municipal wastes and energetic components**

(30) Priority: 07.12.2007 PL 38399007
(71) Applicant: Rogut, Stanislaw, 74-200 Pyrzyce (PL)
(72) Inventor: Rogut, Stanislaw, 74-200 Pyrzyce (PL)
(74) Representative: Kachnic, Tadeusz

(57) **Abstract**

The subject of the invention is the method and processing system especially for municipal wastes and energetic fuel components production.

The method comprises that after the wastes are released from the wrappings, oversized and difficult to grind items are removed and remaining wastes are grinded into pieces smaller than 30 mm and are subject to homogenization process. The waste stream is subject of sterilization process in temperature from 70°C to 90°C and internal drying process in temperature from 90 to 500°C by adding reacting substance. At the same time in reaction with hydration products odours created or emitted in said process are bonded. Received dried product is separated into fractions with various properties, optimally inter alia into ferromagnetic, non-ferrous, fuel, light and heavy mineral fractions.

## Description

The subject of the invention is method and municipal wastes and sewage sludge processing system, and production of energetic fuel components from processed waste.

Known methods of recycling fuels and raw materials from municipal wastes are concentrating on useful ingredients separation contained in wastes, suitable to be re-used. The basic problem in existing separating and sorting technologies from materials, especially waste materials is in useful ingredients efficient separation from wastes into fractions suitable to be recycled. Particular difficulty is in separation of sticky waste materials with high humidity, containing fats and oils with wide diversity of ingredients and form.

The most efficient and widely practiced method of separating waste materials is selective collection. The advantage of such method is recycling and than possibility of re-use separated materials, the disadvantage is separation into pure component raw materials. One of the basic municipal wastes type are wrapping and packing wastes. The technological improvement in packing industry, striving to create wrapping with individual features, adjusted to properties of preserved materials and competition among wrapping producers caused, that only insignificant part of packing wastes are made of homogeneous materials with construction and composition enabling easy waste component segregation.

Present wrapping products are made in complex forms and constructions concluding various materials. The classical example of such wrappings are so called multi-material wrappings, called tetra pack and PET bottles. Common trend to recycle materials concluded in those wrappings is in withdrawal, with regard to fact, that in wrappings are included various construction elements made of different materials or metal (polythene bottles with PCV caps, multilayer wrappings made of polypropylene foil at one side, metalized aluminium foil at the other, and carton and mineral filler between layers and complicated polyvinyl chloride cap). Until now no easy, effective and cheap method of segregation of the materials concluded in those products, into clean and suitable to recycle component materials has been found. Similar problem is with metallic wrappings, especially made of laminated steel plate, and multilayer foils made of aluminium and material or aluminium only. Manual separation of wastes wrappings into particular types of raw materials in case of multilayer wrappings or with constructions concluded various materials will not solve the problem. Selective collecting is not an issue as in this case, much more difficult problem is in purifying metal, plastic or multilayer wrapping elements from remains of products that they preserved, in particular if such materials are containing also residues of fat, meat, animal by-products , oils, varnishes, paints etc.
As a consequence of more complex construction of wrapping materials the quality of recycled materials obtained by separation processes deteriorates and creates difficulties with finding buyers for such materials. More efficient, from economical and environmental point of view, will be in future producing products from original raw materials. The main argument in favour of recycling materials from municipal wastes, especially from wrapping and waste paper, is belief that due to such action we will economize natural raw materials resources. Analysis of product life cycle from excavation of raw materials through production to neutralization of resultant wastes (Product Life Cycle Concept), shows that more environmental friendly is production of paper, wrapping materials and wrappings made of original raw materials rather than made of recycled materials recovered from wastes. Much more economically and environmentally beneficial is processing municipal wastes, especially wrapping, including paper waste into fuel, fuel components and raw materials used especially in cement industry.

Alternative fuel production technologies (RDF - Refuse Delivered Fuels) are relatively advanced, but increase in scale of such fuels production caused problems with their effective use. The most efficient method is co-combustion in cement kilns or combustion in waste incinerator. Scale of possibilities of combusting such fuels in cement plants is relatively small, disproportionate to already existing and prospective possible scale of RDF fuels production. Waste combustion in waste incinerator is very expensive and usually means wasting most of the energy created in those waste incinerating installations. The solution to this problem would be combustion of produced fuels in typical energetic boilers. Fuels recovered from wastes are not very attractive for energetic purposes, due to the form, physicochemical properties and way in which they combusted. Due to large dimensions of particles, high capacity of highly fusible plastics and low volatility in preliminary phases of combustion process in conditions existing in energetic boilers, common phenomenon is an incomplete combustion, resulting with producing product causing operational difficulties, such as covering heat exchange surfaces with sticky products of incomplete combustion, corrosive phenomena and emission of hazardous combustion products. The key to effective, complete and safe combustion of those fuels is to shape them into fine-grained dusts. Traditional methods of crumbling and grinding in case of those fuels fail, due to plasticization of the enclosed in fuel materials, in particular under the influence of the grinding heat, resulting in agglomerating again particles of grinded fuel particles after leaving grinding units. Another barrier of development of such use of fuels and fuel components made of municipal wastes, especially wrappings, in particular multi-material wrappings, is lack of effective and environmental friendly methods of drying before grinding into powdered products and giving produced powder features hindering agglomeration into greater particles during combustion process.

The solution to this problem, according to patent specification No. 4551600, is to dry wastes simultaneously with giving them brittleness features enabling efficient grinding, for example by preliminary carbonisation with heat of part of the exhaust gases, created during preliminary phases of pyrolisis.

Another solution, known from Polish patent specification No. 186862 and from American patent application No. US 20010025448, is to expose crushed and grinded wastes mixed optimally with calcium oxide, to high pressure followed by expansion, to dry them with simultaneous partial pyrolisis. Similar solutions of above mentioned problems are also shown in Polish patent applications No. P-310695, P-313001, P-316406 and Polish patent specifications No. 169195 and 176862.
Most of mentioned solutions are burden with defects of necessity to clean great amount of gases emitted during process and conducting processes in extremely hazardous conditions, threatening with explosion or spontaneous combustion. Produced fuels have low combustion value, since manufactured goods are deprived great amount of energetic ingredients. The other defect is necessity of use complicated and expensive technological appliances. American patent specification No. 6063237 proposes to dry wastes in alkaline environment, in high temperatures, so disintegration takes place as a result of weakening and disintegration of structures bonding various elements of waste components. We can find similar technological solutions in American patent specifications No. 5866754; 51220448 and in American patent application No. US 20040192980.

Another problem occurring during combustion of produced fuels is necessity of effective purification a produced combustion gases from harmful combustion products, such as hydrogen chloride - PCV combustion product, sulphur oxide, fluorine compounds and many more.

The purpose of the invention is to develop method and wastes processing system and production of fuel components, combusted with energetic fuel in energetic boilers.

The purpose of wastes processing, in particular municipal wastes according to the invention is characterized by the fact, that wastes after release from wrappings or bags and oversized and difficult to grind items remove are grinded into pieces smaller than 30 mm and than are subject of homogenization process. Afterwards the waste stream is subject of sterilization process in temperature from 70 °C to 90 °C and internal drying process in temperature from 90 to 500 °C by adding reacting substance reacting with water contained in wastes. At the same time in reaction with hydration products are bonded odours or pungent substances created or emitted from wastes or in said process. Received dried product is separated into fractions with various properties, optimally inter alia into ferromagnetic, non-ferrous, fuel, light and heavy mineral fractions.Fuel fractions are grinded along with mineral elements.

As mineral elements added to fuel fractions are use separately or jointly in various combinations: burnt lime, hydrate lime, calcium carbonate, semi-hydrated gypsum, coal sludge, mud-form carbon, and by-products of processing condensed sewage sludge and/or animal raw materials and/or biomass.

Wastes grinding process is performed in preliminar grinding phase into pieces smaller than 50 mm and in final grinding phase, into pieces smaller than 30 mm.

Grinded waste streams go through drying and homogenization process, performed continuously until the drying process is finished.

The best reacting substances are metallic oxides of chemical elements of groups I to III of periodic table.

The reacting substance in sterilization and drying process is preferably high reactivity grinded burnt lime with optimal parameter t₆₀ < 2 minutes.

The internal drying process is performed by passing through ingoing wastes stream an overheated dry steam with temperature of 100 to 500 °C created automatically inside the stream of reacting wastes as a result of exothermal energy generated during hydration process between reagent and water present in waste.

Sterilization process is performed by heating the components with exothermal energy heat created during the process, in sterilization temperature above 70°C.

Process of bonding the odours is performed by bonding odour substances with metallic hydrates created in the process, to receive low solubility chemical compounds.

The method and processing system especially for municipal wastes and energetic fuel components according to the invention is characterized by containing waste collection module, oversized elements selection module, grinding module , homogenization module, reacting substance dosage module, sterilization and drying module with exothermal chemical heat generation module , separation of products module especially into ferromagnetic, non-ferrous, fuel, mineral and organic fractions. Furthermore the system contains fuel fraction grinding module, connected with module completing received product with mineral substances acquired from outside sources, and used in co-grinding with fuel fractions to form defined final fuels or fuel additives with dedicated properties.

According to the invention, the energetic fuel component containing fuel fractions is characterized by containing flammable substances, substances bonding impurities created during burning processes , lowering combustion temperature, and reducing content of nitric oxides emitted during energetic fuel combustion process.

According to the invention, the component optimal content is grinded fuel fractions and products received from processing sewage sludge in 10 to 50 %, and optimally 30% by weight.

The component, according to the invention, should be fuel with content of 10 to 40% by weight of calcium hydrate and added in 10 to 40% parts by weight to energetic fuels with high content of sulphur and chlorine compounds, to bond created during combustion process harmful chemical compounds.

The component is added directly to the boiler, optimally with fluidized bed or grinded with other types of fuels.

According to the invention, the method consist in dispatching municipal wastes in plastic bags and containers from household or catering, unloading them to unloading facilities, and then transporting them with conveyor to oversized items removing facilities. After oversized elements automatic removal from waste stream, wastes are carried to grinding appliances in grinding and homogenization module. Grinding appliances in grinding module are selected to allow grind the products into pieces smaller than 10 mm, enabling drying them with FuelCal^{®} technology. The FuelCal^{®} technology consist in mixing vertically moving stream of wet materials with very high reactivity grinded burnt lime with simultaneous displacing received mixture down into a vertical adiabatic reactor, in intensively exothermic hydration reaction conditions, so the emitted hydration thermal energy heats up part of moisture contained in reactive mass, into overheated steam, said steam directed upwards against the stream of reactive components causes drying and sterilization of the aforementioned reactive mass.

Grinded and preliminarily homogenized mixture of wet wastes, with natural humidity approximately of 30%, as homogeneous stream is carried into waste sterilizing and drying system inlet according to FuelCal^{®} technology. Vapours emitted during drying and sterilization are condensed, and recovered during condensation heat is used to support drying process. At the end of FuelCal^{®} technological line the reacting mass of components containing sterilized, grinded and dried wastes and created from used in the process reacting substance - calcium hydrate obtains features enabling effective segregation into particular components.
Grinded and dried products stream goes to metal elements separation module, where elements containing iron and non-ferrous metals are separated. Stream of the product without metallic elements are separated into fractions with various dimensions and density. Separation process use sieve separators, where dried products are distributed into fractions differ in dimensions, air separators where separation takes place according to material density, and inertial separators. Received material fractions are than processed according to their final use. Fractions with the highest content of flammable components, such as sterilized waste paper, multilayer wrappings, wood, plastics, dried pieces of biomass containing from 5 to 25% of calcium hydrate, are processed into fuel or fuel components. For this reason the fuel components are transported to chain, cyclone or turbo mills. Dry powdered fuels are made of grinded material with calcium hydrate formed in sterilization and drying process and mineral components present in wastes purposely added from outside to the grinded material. In dry powdered fuels, fuel particles are enclosed with dust mineral products, preventing lumping and agglomerating phenomenon, in grinding process as well as during storage, transportation and combustion.

The unexpected result of the process, executed according to the invention is that calcium hydrate granules occurring in fuel particles structures give to the produced fuel, new unique properties of bonding harmful gas pollutants in particular chlorine, phosphorus, sulphur and fluorine compounds created during combustion. Calcium hydrate present in product, after reaching during combustion thermal hydrate disintegration temperature of 520-530 °C, generates reactive particles of calcium oxide, bonding harmful gas pollutants created during combustion process.
Additional, unexpected result of conducting the process according to the invention is that calcium hydrate in combusted product during endothermic reaction of thermal disintegration in temperature of 520 -530 °C, absorbs from the reacting environment part of the combustion heat, causing cooling of the created flame. It is especially significant in energetic boilers, combusting highly caloric fuels in fluidized bed, as it leads to lowering maximal combustion temperature and in consequence to limitation of produced nitric oxides and extension of operating time of boiler construction elements, exposed to erosion in high temperature environment. Phenomenon of lowering combustion temperature in energetic boilers is possible only in case of using fuel components produced according to the invention and containing calcium hydrate. Flame cooling effect cannot be achieved when using calcium carbonate in fluidized bed boilers as desulfurization element. The endothermic process of calcium carbonate disintegration takes place in higher temperatures corresponding to thermal disintegration of calcium carbonate, that is 890 - 920 ° C.

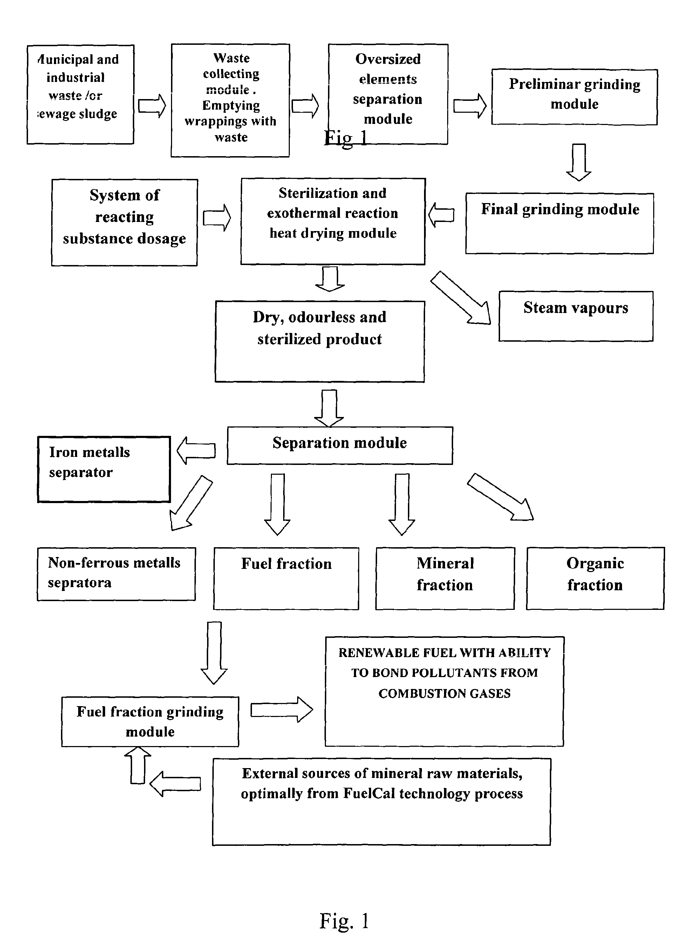

Schematic method of process implementation according to the invention is shown on Fig. 1.

### Example 1

Processing wastes into fuels and fuel components witch method according to the invention consist in raw municipal wastes, containing on average 40 % by weight of humidity, 10% of mineral components, including 6% of glass, 6% of metallic waste, 24% of organic components; 20% of wrapping wastes including 6% of plastics, 8% of waste paper, 6% of multi layer wrapping wastes with average heat value of 5000 kJ/kg, in quantity of 10 Mg/h are transferred from unloading container to belt conveyor where bags are opened and than transported to oversized elements separation module, to remove the elements that due to dimension or construction could damage grinding appliances. After removing dangerous elements, waste stream is dispatched into preliminar grinding module, where it is grinded to pieces below 50mm, and then into final grinding module where is grinded into pieces smaller than 30mm. Grinded wastes in the form of wet and sticky mass are delivered to the inlet of sterilizing and drying module, where due to exothermal reaction heat undergo thermo -chemical sterilization and drying processing . Process is performed according to the FuelCal^{®} technology, consisting in mixing vertically situated stream of damped materials with grinded burnt lime with very high reactivity, with simultaneous displacing received mixture down into a vertical adiabatic reactor, in intensively exothermic hydration reaction conditions, so the emitted hydration thermal energy heats up part of moisture contained in reactive mass, into overheated steam, said steam directed upwards against the stream of processed components causes drying and sterilization of tem. In reactor the wastes are mixed with reacting substance best with grinded burnt lime with high reactivity in 20% by weight with parameter t₆₀ characterized by reactivity of t₆₀ < 2 minutes. As a result of proceeded chemical hydration and neutralization reactions, grinded wastes are chemically and thermally sterilized and than dried to form of loose product containing no more than 16% of humidity, enabling effective components separation. Product in final phase of chemical reaction leaves reactor and goes into metal separation module, where using magnetic separators elements with ferromagnetic properties are separated, mainly ferrous metals and steel, and then with eddy current separators non-ferrous metal elements are separated, mainly aluminium and copper. Leaving metal separation module, the dried product stream free from metal elements, goes to sieve separators system, where is separated into fuel fraction with dimensions of 30 to 10 mm and mineral-organic fraction with dimension less than 10 mm. As a result of performed processes, wastes are separated into several fractions: oversized wastes for separate utilization, scrap iron and steel scrap fraction, non-ferrous scrap fraction, fuel fraction and mineral-organic fraction. Fuel fraction containing mainly grinded and preliminary dried waste paper, multi material wrappings and plastics, larger particles of sterilized and dried biomass, part of calcium hydrate and glass particles created during the process, in total amount of 30% by weight of crude waste and reacting substance, are directed into chain mill inlet, where they undergo further grinding and drying process with grinding heat and continued chemical reaction, and are grinded into form of powdered fuel with granulation best below 1mm. As a result of the process we receive fuel component in form of renewable powdered fuel in approximately 25% by weight of total amount of processed wastes and reacting substance. Product with average heating value of 18 MJ/kg contains up to 20% by weight of calcium hydrate. It has ability to bond gas pollutants emitted during fuels combustion. Fuel component by virtue of received structure and chemical composition can be burnt in typical energetic boilers.

### Example 2

Wastes processing process is performed according to example 1 with the difference, that to received in separation fuel fraction, before grinding, to stimulate drying and grinding process and to increase created product desulphurization abilities, grinded burnt lime is added in 10% by weight.

### Example 3

Wastes processing process is performed according to example 1 with the difference, that fuel fraction received in separation, containing 16% of moisture, with average heating value of 24 GJ/kg, before going through grinding process, is mixed with product of processing condensed sewage sludge by FuelCal^{®} technology, containing 25 % of moisture, 35% of organic components, 30% of calcium hydrate, 10% of mineral components with average heating value of 10 GJ/kg in 30% by weight. Received mixture is grinded in chain mill, until the particles receive dimension less than 1 mm. As a result of simultaneous grinding and drying operation fuel component is received with average humidity of 4%, containing approximately 25% of calcium hydrate with ability to desulfurizate combustion gases and with average heating value not less than 20 GJ/kg. Through co-grinding communal waste fuel fraction with sewage sludge processing product containing high content of calcium hydrate, in produced fuel component increases content of elements able to desulphurise combustion gases, tendency of fuel particles to agglomerate during transportation and storage is reduced and maximal combustion temperature in energetic boilers, in particular fluidized boilers is decreased.

### Example 4

Wastes processing process is performed according to example 1 with the difference, that separated fuel fraction containing 5% of calcium hydrate is mixed before grinding in proportions 50%:50% by weight with waste carbon sludge containing 15% of calcium hydrate, dried with FuelCal^{®} technology, and grinded in coal mill in order to achieve better product disintegration and drying. Produced fuel component, containing approximately 10% of calcium hydrate, free from tendencies to agglomerate and paste during combustion, is added in 30% by weight as fuel component additive to fuel to the energetic boiler with fluidized bed, in order to desulfurize combustion gases and to lower maximal combustion temperature by approximately 30°C, which reduces erosion processes in boiler.

### Example 5

Wastes processing system is characterized by delivering wastes to the wastes collecting module where bags or containers with wastes are automatically emptied. During wastes transmission on conveyor belt, before sending them to preliminary and final grinding module, oversized or having properties that could damage grinding appliances wastes are removed with automated separating appliances, optimally with remotely controlled grasper. In preliminar grinding module wastes are grinded into pieces below than 50 mm, and in final grinding module into pieces less than 30 mm. Equal moisture content and uniform waste mixture is obtained as a result of homogenization during grinding process. Received mixture goes into sterilization and drying module, where due to exothermic reaction, proceeded between reacting substance best grinded burnt lime with high reactivity and components present in wastes, in particular with water and acid compounds, wastes are sterilized and dried and odour substances present in mixture are chemically bonded into low solubility and volatility chemical compounds. During the process, as a result of grinding and trituration phenomena further homogenization of mixture components, occurs forming dry, odourless and sterilized product, enabling effective separation into particular fractions.

Received product goes to separation module, where it is divided into: ferromagnetic metal fraction - ferrous metals; non-ferrous metal fraction - mainly aluminium and copper; fuel fraction - mainly plastics, waste paper and wrapping wastes and larger dried and sterilized biomass particles enclosed with mineral components, mainly with calcium hydrate; mineral fraction - mainly crumbled glass from glass bottles and containers and mineral components such as sand and detergents used in households,; organic fraction - mainly crumbled and dried biomass, especially from catering and kitchen wastes.

Fuel fraction with the highest heating value goes to fuel fraction grinding module , where it is grinded into fine-grained fuel or fuel component with optimal dimensions below 1 mm. To optimise functional properties of created product, the fuel fraction before grinding, is completed with elements containing mineral components with ability to bond pollutants from combustion process and preventing grinded particles agglomeration. Most often used additional elements are sewage sludge and biomass products received from FuelCal^{®} technology and dry sludge coal , optimally dried with reactive burnt lime.

### Example 6

The energetic fuel component produced according example 1, with the difference, that before grinding to fuel fraction is added mineral fraction received during separation. Received in grinding process product is than used as corrective raw material kiln feedstock in clinker burning installations.

### Example 7

The fuel component produced according example 1, with the difference, that before grinding to the fuel fraction isolated in the process mineral fraction is added in 100 % by weight containing 30% of calcium hydrate or additional mineral element from sewage sludge processing or animal by-products with similar content of calcium hydrate. Received products containing on average 20% of calcium hydrate is added in 30% by weight as energetic fuel component, combusted in fluidized boilers to bond pollutants in combustion gases and to lower flame maximal temperature in fluidized boiler, reducing the quantity of generated nitric oxides and extending the operation of energetic appliances.

### Example 8

The energetic fuel component produced according to example 7, with the difference, that before grinding to fuel fraction, instead of isolated mineral fraction in 100% by weight is added sewage sludge processing product or animal by-products with similar content of calcium hydrate.

### Example 9

The energetic fuel component produced according example 1, 7 or 8 with the difference, that it is added as additional element to the energetic fuel before grinding or directly to grinded fuels stream injected into the boiler.

## Claims

1. Wastes processing method, especially processing municipal wastes into secondary materials or semi-products, consisting in recycling raw materials in segregation process, is **characterized by** the fact, that after releasing wastes from transport containers , oversized and difficult to grind items are removed from the wrappings, and remaining wastes are grinded into pieces less than 30 mm and homogenised, and then waste stream goes through sterilization process in temperature from70°C to 90°C and internal drying process in temperature from 90 to 500°C by introduction reacting substance, reacting with water in wastes. At the same time during the reaction with products created in hydration process odours created or emitted in performed process are bonded, and received dried product is separated into fractions with various properties, optimally alter alia into ferromagnetic fractions, non-ferrous, fuel, light and heavy mineral fractions.

2. Method according to claim 1, wherein the fuel fractions goes through grinding process along with additional mineral components.

3. Method according to claim 1, wherein as additional mineral elements to fuel fractions are used separately or jointly in various combinations: burnt lime, hydrated lime, calcium carbonate, semi- hydrated gypsum, sludge -form carbon, optimally as products of condensated waste sludges processing and/or animal raw materials and/or biomass.

4. Method according to claim 1, wherein the waste grinding process is performed in preliminar grinding phase into pieces smaller than 50mm and in final grinding phase, into pieces smaller than 30mm.

5. Method according to claim 1, wherein the grinded waste streams go through homogenization process, performed in continuous way until the drying process is finished.

6. Method according to claim 1, wherein reacting substances are optimally metallic oxides of chemical elements of groups I to III of periodic table.

7. Method according to claim 1, wherein the reacting substance used in sterilization and drying process is grinded burnt lime with increased extent of reactivity, with optimal parameter t₆₀ < 2 minutes.

8. Method according to claim 1, wherein the internal drying process is performed by passing through a stream of ingoing waste overheated with dry steam with temperature of 100 to 500°C created automatically inside the stream of reacting waste, as a result of released exothermal hydration energy.

9. Method according to claim 1, wherein the sterilization process is performed by heating the components with exothermal energy heat created during the process, in temperature above 70°C.

10. Method according to claim 1, wherein the process of bonding the odours is performed by bonding odour substances with metallic hydrates created in the process, to receive low solubility and volatility chemical compounds.

11. Wastes processing system, especially municipal wastes, wherein containing waste collecting modules, oversized elements selection, grinding, homogenization, reacting substance dosage modules, sterilization and drying with exothermal chemical reaction heat, separation of product module, especially into ferromagnetic, non-ferrous, fuel, mineral and organic fractions, and fuel fraction grinding module connected with module completing with mineral substances, acquired from outside sources, and used in co-grinding with fuel fractions to form products defined composition and parameters.

12. An energetic fuel component containing fuel fractions, wherein containing flammable substances, substances bonding pollutants and lowering combustion flame temperature, and reducing content of nitric oxides in waste gases emitted during energetic fuel combustion process.

13. The component according to claim **12,** wherein containing grinded fuel fractions and products received from processing sewage sludge in 10 to 50 %, and optimally 30% by weight.

14. The component, according to claim **12,** wherein the fuel fraction containing 10 to 40% by weight of calcium hydrate form 10 to 40% parts by weight of additional components of energetic fuels with high content of sulphur and chlorine compounds, to bond created in combustion process harmful chemical compounds.

15. The component according to claim **12,** wherein is added directly to the boiler, optimally with fluidized bed or grinded with other types of fuels.
